# EUROPÄISCHE PATENTSCHRIFT

(11) **EP 3 993 722 B1**
(45) Veröffentlichungstag und Bekanntmachung des Hinweises auf die Patenterteilung: **21.08.2024**
(21) Anmeldenummer: 20746643.4
(22) Anmeldetag: 27.07.2020
(51) Int. Cl.: A61B 17/70

(54) **VERBINDUNGSEINRICHTUNG FÜR WIRBELSÄULENSTÜTZE**
CONNECTOR FOR SPINAL COLUMN SUPPORT
CONNECTEUR POUR SUPPORT DE COLONNE VERTÉBRALE

(30) Priorität: 30.07.2019 DE 102019005376
(43) Veröffentlichungstag der Anmeldung: 11.05.2022
(73) Patentinhaber: Signus Medizintechnik GmbH, 63755 Alzenau (DE)
(72) Erfinder: VAN DER POL, Bas, 63755 Alzenau (DE)
(74) Vertreter: Leinweber & Zimmermann
(86) Internationale Anmeldenummer: PCT/EP2020/071154
(87) Internationale Veröffentlichungsnummer: WO 2021/018846

(56) Entgegenhaltungen:
- EP-A2- 2 480 149
- US-A1- 2012 109 202
- US-A1- 2014 277 163
- US-B1- 6 368 320

## Beschreibung

Die Erfindung betrifft eine Verbindungseinrichtung zur Verbindung zweier längs eines Wirbelsäulenabschnitts gelegten oder zu legenden Stützstangen einer Wirbelsäulenstützvorrichtung, mit einer Querstange, einer die Querstange mit einer ersten der Stützstangen koppelnden ersten Kopplung und einer die Querstange mit der zweiten der Stützstangen koppelnden zweiten Kopplung, bei der wenigstens eine der Kopplungen einen die Stützstange mit einer Klemmkraft einklemmenden Klemmbereich und Kraftbeaufschlagungsmittel zur Erzeugung einer die Klemmkraft bewirkenden Axialkraft aufweist, insbesondere in Form einer axialen Spannschraube, durch deren Einschrauben die Axialkraft bedingt wird.

Derartige Verbindungseinrichtungen sind gut bekannt, beispielsweise aus der US 2007/0049932 A1. Bei den darin gelehrten Verbindungseinrichtungen wird durch das Einschrauben der Spannschraube nicht nur die die Stützstange einklemmende Klemmkraft bewirkt, sondern die Spannschraube ist noch von einer Spreizhülse umgeben, die sich beim Einschrauben radial aufspreizt und dadurch die Querstange in einer Querstangenrinne ebenfalls einklemmt.

US 6,368,320 B1, US 2012/0109202 A1 und US 2014/0277163 A1 offenbaren jeweils eine Verbindungseinrichtung mit den Merkmalen des Oberbegriffs von Anspruch 1.

Der Erfindung liegt die Aufgabe zugrunde, eine Verbindungseinrichtung der eingangs genannten Art insbesondere im Hinblick auf eine zufriedenstellende Kombination aus einfacher Struktur der Verbindungseinrichtung und ihrer einfachen Handhabbarkeit weiterzubilden.

Diese Aufgabe wird von der Erfindung durch eine Weiterbildung der Verbindungseinrichtung der eingangs genannten Art mit den Merkmalen von Anspruch 1 gelöst.

Einerseits ist somit vorgesehen, dass die Axialkraft durch die Querstange geleitet wird. Die Querstange ist somit Teil des Kraftübertragungswegs, auf dem ausgehend vom etwa Einschrauben bis zum Festziehen der Spannschraube das kraftbeaufschlagte Einklemmen bzw. die Herstellung einer starren Kopplung zwischen Verbindungsstange und Querstange bewirkt wird.

Dies erleichtert die Handhabung und die Verbindungseinrichtung kommt mit wenigen Bauteilen aus.

Andererseits weist ein erster Teil des Klemmbereichs einen federnden Arm auf. In diesem Zusammenhang kann der Klemmbereich auf die Stützstange aufclipsbar sein, indem der federnde Arm zurückweicht und aufgrund seiner federnden Lagerung in seine Ursprungslage drängt. Es versteht sich, dass das Aufclipsen bevorzugt werkzeugfrei von Hand möglich ist.

Bevorzugt ist der Klemmbereich einstückig und insbesondere einstückig mit der Kopplung abgesehen von dem Kraftbeaufschlagungsmittel (z.B. einer Spannschraube) gebildet. Dabei wird besonders bevorzugt vorgesehen, dass eine federnde Lagerung des Federarms durch eine Materialschwächung der Kopplung gefördert ist. Letztere kann z.B. durch eine durch den Körper der Kopplung gehende Bohrung gebildet sein. Dies erleichtert die Herstellung bei möglichst wenig Bauteilen.

Erfindungsgemäß liegt eine auf der der Spannschraube abgewandten Seite der Querstange gebildete Auflage für die Querstange, ohne Beaufschlagung durch die Axialkraft, im Bereich des federnden Arms axial auf einem höheren Niveau als in einem anderen Teil des Klemmbereichs. Auf diese Weise gelingt eine besonders einfache Kraftbeaufschlagung des federnden Arms. Durch die Kraftbeaufschlagung wird die federnde Wirkung des Arms aufgehoben und bei ausreichender Axialkraft eine zur starren Kopplung ausreichende, radial auf die Querstange wirkende Klemmkraft hervorgerufen. Hierzu sind bevorzugt die der Stützstange zugewandten Klemmflächen des Klemmbereichs im Querschnitt gesehen wenigstens teilweise komplementär zur Kontur der Stützstange ausgebildet.

Die federnde Lagerung, insbesondere die diese bewirkende Materialschwächung kann, im Axialschnitt gesehen, bezüglich der Spannschraube asymmetrisch lokalisiert sein, bevorzugt zur Seite des Federarms hin. Dies erleichtert die Abstimmung der Verformungswege und vermeidet überlange Verformungswege am freien Ende des Federarms.

Die Verbindungseinrichtung kann bevorzugt mehrere Zustände einnehmen, etwa einen Zustand, in dem die Querstange in den Kopplungen bereits gehalten, jedoch noch eine Axialverschieblichkeit entlang der Querstange zur Einstellung eines Positionierabstands möglich ist, einen Zustand, in dem der Positionierabstand fest eingestellt ist und auch eine radiale Kopplung zu den Stützstangen bereits besteht, jedoch noch ein Verschieben entlang der Stützstangen möglich ist, wie auch den zuletzt angestrebten Endzustand der starren Kopplung der beiden Stützstangen miteinander durch die Verbindungseinrichtung. Letzteres wird im einfachsten Fall einer Realisierung der Kraftbeaufschlagungsmittel durch das Festziehen einer Spannschraube erreicht, die vorhergehenden Zustände entsprechen Zuständen niedriger Einschraubtiefe/Drehmomentbeaufschlagung der Spannschraube.

Die Kopplungen können ähnlich bis identisch ausgeführt sein (dennoch ist bei der Montage eine nicht identische Gestaltung möglich, indem beispielsweise die jeweiligen Federarme der Kopplungen voneinander weggewandt sind). In einer sehr einfachen Gestaltung kann die Verbindungseinrichtung nur aus fünf Bauteilen insgesamt und drei unterschiedlichen Bauteilen insgesamt bestehen (zwei identische Aufnahmen, zwei identische Spannschrauben und die Querstange). Es könnte jedoch auch vorgesehen sein, dass die Querstange und die andere Kopplung bereits fest verbunden sind und als ein Bauteil, z.B. über eine Rinne und Feststellschrauben an einer in die Rinne eingelegte Stützstange befestigt wird.

In einer bevorzugten Ausführungsform weisen beide Kopplungen jeweils eine Aufnahme auf, die an ihrer in Axialrichtung oberen Seite einen Einschnitt aufweist, an deren unterem Endbereich ein Aufnahmeraum in Form einer in Querrichtung durchgehenden Rinne gebildet ist. Weiter bevorzugt weisen sich entlang des Einschnitts in Axialrichtung erstreckende Seitenbereiche an ihrer Innenseite ein Gewinde auf, mit dem die axiale Spannschraube zusammenwirkt.

Die Erfindung stellt jedoch auch Sortimente mit z.B. Querstangen unterschiedlicher Länge bereit, oder auch Kopplungen, die zur Ankopplung an Stützstangen unterschiedlichen Durchmessers ausgelegt sind.

Die Verbindungseinrichtung eignet sich somit sowohl für neu zu implantierende Wirbelsäulenstützen, wie auch als zusätzliche Querversteifungen für bereits bestehende (bei denen je nach Patient und Systemhersteller bzw. Anforderungen der damaligen Implantierung unterschiedliche Querabmessungen der Stützstangen bestehen können).

Die Erfindung stellt auch eine Kopplung einer solchen Verbindungseinrichtung als solche unter Schutz, wie auch das Herrichten einer solchen Verbindungseinrichtung für ihren bevorstehenden Einsatz der Schaffung einer Querverbindung zwischen zwei Stützstangen einer Wirbelsäulenstützvorrichtung. Diese kann mehrere Aspekte beinhalten, zum einen die (ohnehin vorgesehene) Ausgestaltung der Verbindungseinrichtung materialtechnisch aus biokompatiblen Materialien (wie etwa Titan, rostfreier Stahl, oder auch Kunststoffe, etwa biokompatibler Polymere, die dem Fachmann aus dem Gebiet der Implantologie bekannt sind), wie auch die sinnfällige Herrichtung durch Desinfektionsschritte wie z.B. Autoklavieren, die passende Zusammenstellung passend zu Stützstangen der Wirbelsäulenstützvorrichtung und/oder die griffbereite Bereitlegung der zusammengestellten Teile für den das Implantieren vornehmenden Chirurgen.

Des Weiteren stellt die Erfindung auch ganze Wirbelsäulenstützvorrichtungen mit derartigen Verbindungseinrichtungen unter Schutz. Dabei können die Wirbelsäulenstützvorrichtungen neben den Stützstangen auch die Anbringungsmechanismen für die Stützstangen umfassen, etwa Pedikelschrauben jeglicher Gestaltung und deren Kopplungsmechanismen an die mehrere Pedikelschrauben starr verbindende Stützstange über beispielsweise polyaxiale Einstellungen erlaubende Verbindungselemente (feste Kopplung in polyaxial ausbildbarer Winkelanordnung der Pedikelschraube zur Stützstange).

In einer einfachen Gestaltung, bei der man von annähernd parallelen zu verbindenden Stützstangen ausgeht, kann deren Erstreckungsrichtung, die der Verlauf der Querstange sowie die Axialkraft (axiale Einführrichtung der Spannschrauben) ein rechtwinkliges Koordinatensystem festlegen, es versteht sich jedoch, dass auch nicht orthogonale Querstellungen möglich sind oder auch einstellbare Winkelanordnungen, letzteres mag jedoch die Anzahl der minimal erforderlichen Bauteile erhöhen.

Weitere Merkmale, Einzelheiten und Vorteile der Erfindung ergeben sich aus der nachfolgenden Beschreibung mit Bezug auf die beigefügten Figuren, von denen
Fig. 1 einen Teil einer Kopplung einer Verbindungseinrichtung zeigt, und zwar
Fig. 1a in einer Vorderansicht mit Blickrichtung in Verbindungsrichtung,
Fig. 1b in einer Seitenansicht mit Blickrichtung der Stützstangenerstreckung, und
Fig. 1c in einer Schnittansicht orthogonal zur Blickrichtung der Fig. 1b,
Fig. 2 eine Verbindungseinrichtung zeigt, und zwar in Fig. 2a ohne und in Fig. 2b mit Spannschrauben, und
Fig. 3 eine Schnittansicht von Fig. 2b mit eingezeichneten Kraftpfeilen ist.

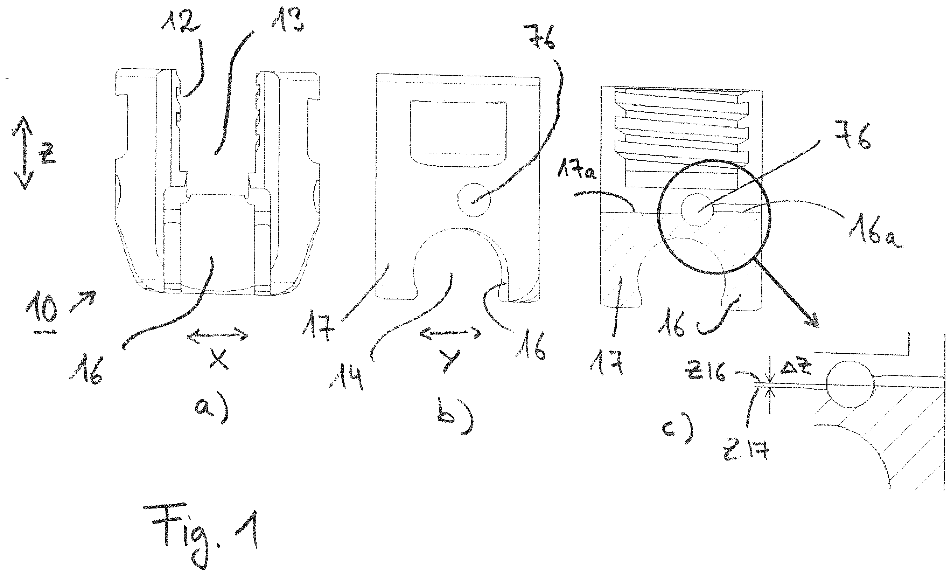

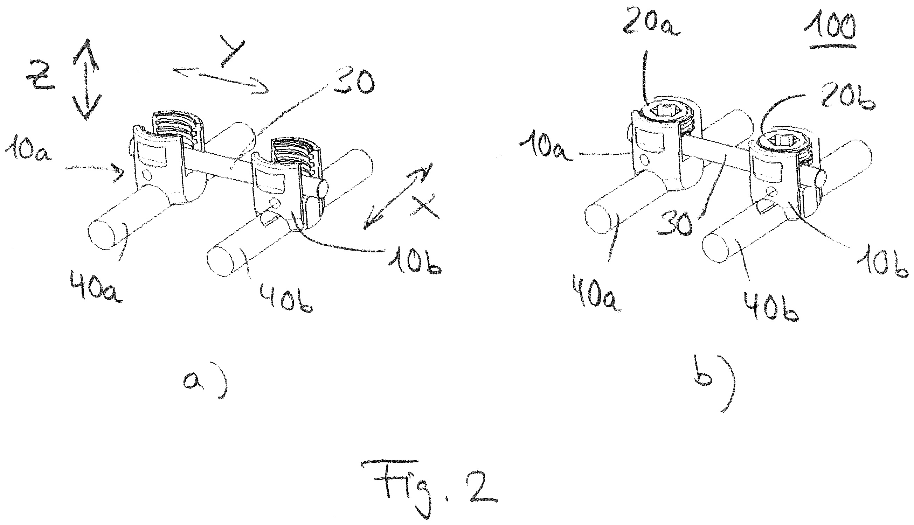

In Fig. 1 ist eine Aufnahme 10 (hierin auch Kopplung genannt) dargestellt, die, zusammen mit einer in Fig. 1 nicht dargestellten, mit dem Gewinde 12 der Aufnahme 10 zusammenwirkende Spannschraube in jeweils doppelter Ausführung zusammen mit einer Querstange 30 eine in Fig. 2b zusammenwirkend dargestellte Verbindungseinrichtung 100 bildet, welche zwei Stützstangen 40a, 40b im Wege einer Querversteifung miteinander verbindet.

Obwohl dies in Fig. 2b nicht dargestellt ist, sind die Stützstangen 40a, 40b Teile einer Wirbelsäulenstützvorrichtung, und selbst mit in Wirbelkörper eingeschraubten Pedikelschrauben über hierzu geeignete Kopplungen starr verbunden, wenn im menschlichen Körper implantiert. Derartige Wirbelsäulenstützvorrichtungen sind in der Technik bekannt und es wird daher nicht weitergehend darauf eingegangen, sondern diesbezüglich auf einschlägige Systeme Bezug genommen, wie sie beispielsweise in den durch Bezugnahme eingeschlossenen WO 2009/015100 A2 und EP 2 581 057 B1 offenbart sind.

In der Vorderansicht von Fig. 1a ist erkennbar, dass die Aufnahme 10 an ihrer in Axialrichtung Z oberen Seite einen Einschnitt aufweist, an deren unterem Endbereich ein Aufnahmeraum in Form einer in Y-Richtung durchgehenden Rinne gebildet ist. Wie ebenfalls aus Fig. 1a und Fig. 2a erkennbar ist, kann die Querstange 30 von oben in Axialrichtung entlang des Einschnitts, an dessen unterem Endbereich der Aufnahmeraum für die Querstange gebildet ist, in den Aufnahmeraum eingeführt werden. Die sich entlang des Einschnitts in Axialrichtung erstreckenden Seitenbereiche tragen an ihrer Innenseite das Gewinde 12. Mit Blickrichtung der Axialrichtung Z erscheint der Einschnitt als Gewindebohrung, die von der Rinne 13 in Querrichtung Y durchbrochen ist.

In den bezüglich der Axialrichtung Z unteren Bereich der Aufnahme 10 ist eine quer, in diesem Ausführungsbeispiel orthogonal zur Rinne 13 verlaufende Aufnahmerinne 14 gebildet, die besser in Fig. 1b erkennbar ist. Diese ist dazu ausgelegt, eine in Fig. 1 nicht dargestellte Stützstange 40 aufzunehmen und in Umfangsrichtung um mehr als 180° zu umgeben. Ein bezüglich der Erstreckungsrichtung der Erstreckungsrichtung X der Stützstange 40 mittig angeordneter Federarm 16 bildet zusammen mit einer diesem gegenüberliegenden Seitenhalterung 17 einen Klemmbereich, in dem die Stützstange 40 eingeklemmt und schließlich fest eingespannt wird.

Hierzu ist in einer bevorzugten Ausgestaltung die Engstelle zwischen den einander zugewandten unteren Endabschnitten der Seitenhalterung 17 und des Federarms 16 von geringerem gegenseitigen Abstand als der Durchmesser der Stützstange 40, jedoch nur geringfügig geringer. Der Klemmbereich 16, 17 kann somit auf die Stützstange 40 aufgeclipst werden, wobei sich der Federarm 16 bei der Aufclipsbewegung aufgrund seiner federnden Lagerung elastisch verformt. Die federnde Lagerung im Übergangsbereich zur Seitenhalterung 17 wird gefördert durch eine Materialschwächung 76 in Form einer sich in X-Richtung durch die Aufnahme 10 hindurchgehende Bohrung. Es verbleibt somit eine Materialbrücke zwischen dem in Fig. 1c unteren Rand der Bohrung 76 und der diesem gegenüberliegenden Abschnitt der Begrenzung der Rinne 14.

Ist die Aufnahme 10 auf Stange 40 aufgeclipst, so kann sie umgekehrt auch wieder von der Stange 40 abgenommen werden, es sei denn, die Klemmkraft des Klemmbereichs 16, 17 ist durch Wirkung der in diesem Ausführungsbeispiel durch die Spannschraube 20 gebildeten Kraftbeaufschlagungsmittel so stark, dass das radiale Abheben nicht mehr möglich ist.

In Fig. 1c und ihrem Vergrößerungsanteil ist gut zu erkennen, dass in einem nicht kraftbeaufschlagten Zustand das Höhenniveau Z16 der oberen Fläche 16a des Federarms 16, welche eine Auflagefläche für die Querstange 30 bildet, um einen Höhenunterschied ΔZ in Axialrichtung Z höher liegt als das Höhenniveau Z17 der oberen Fläche 17a der Seitenhalterung 17, die ebenfalls Auflagefläche für die Querstange 30 ist. Bezeichnet der Federarm 16 somit den beweglichen Schenkel und der Seitenhalterung 17 den festen Schenkel des hier realisierten Klemmmechanismus unter axialer Kraftbeaufschlagung, liegt die Auflagefläche 16a des beweglichen Schenkels dem Gewinde 12 in Axialrichtung Z näher als die des festen Schenkels 17.

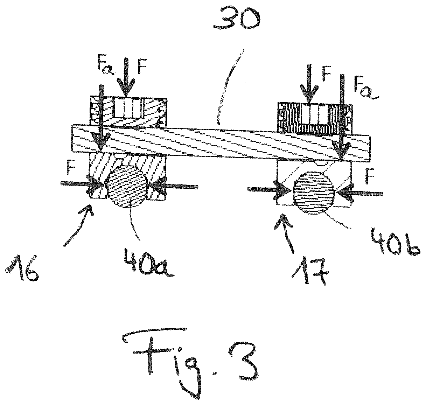

Wird nun die Spannschraube 30 in das Gewinde 12 eingeschraubt, wird die dadurch hervorgerufene Axialkraft durch die Querstange 30 geleitet und wirkt zunächst auf die Auflagefläche des Federarms 16, der hierdurch axial nach unten gedrückt wird, was sich aufgrund der zur Stange 40 komplementären Innenflächen des Klemmbereichs in radialen Querkräften niederschlägt, die in Fig. 3 in Y-Richtung gezeichnet dargestellt sind. Die Innenflächen des Klemmbereichs 16, 17 gelangen dabei in Druckanlage um die Stange 40 über einen größer als 180° betragenden Winkelbereich, bevorzugt größer als 200°, in der vorliegenden Ausführungsform ca. 225°.

Es versteht sich, dass ein Festziehen der Spannschraube 20a (d.h., der Ausübung eines über einer vorgegebenen kritischen Schwelle liegenden Drehmoments) für eine feste starre Kopplung der Stützstange 40 mit der Aufnahme 10 sorgt. Es versteht sich weiterhin, dass aufgrund der konstruktionsgemäßen Gestaltung, die Axialkraft durch die Querstange zu leiten, dabei auch die Querstange 30 starr und fest mit den Aufnahmen 10 gekoppelt wird (dieser Zustand ist in den Figuren 2b und 3 gezeigt).

Im Einsatz könnten beispielsweise die zwei Aufnahmen 10a, 10b (Fig. 2a) der Verbindungseinrichtung 100 separat auf die zu verbindenden Stützstangen 40a, 40b aufgeclipst werden und, wie in Fig. 2a gezeigt, die Querstange 30 in die Aufnahmen 10a, 10b eingeführt werden, bis sie im Querstangenkanal 13 auf den Auflageflächen 16a der jeweiligen Federarme 16 zu liegen kommt. Alternativ zur Gestaltung von Fig. 2 könnte man auch vor dem Aufclipsen der Aufnahmen 10a, 10b diese beiden Aufnahmen mittels der Querstange 30 und den Spannschrauben 20a mit nur sehr geringem, ein Herausrutschen aus dem Kanal 13 in Z-Richtung verhindernden Einschrauben der Spannschrauben 20 miteinander koppeln, so dass der Abstand der beiden Kopplungen noch durch Verschiebung entlang der Achse Y einstellbar ist, diesen Abstand passend zum Abstand der Stützstange 40a, 40b einstellen und durch geringfügig weiteres Einschrauben der Spannschrauben 20a, 20b festlegen. In diesem Zustand können die bereits mit der Querstange 30 verbundenen Kopplungen auch gemeinsam auf die jeweiligen Stützstangen 40a, 40b aufgeclipst werden.

Ein nochmals weiteres Eindrehen verhindert dann ein Lösen der Kopplungen von den Stützstangen 40a, 40b, erlaubt jedoch noch eine entlang deren Erstreckungsrichtung X vorgenommene gemeinsame Verschiebung zur entsprechenden Festlegung der endgültigen Position, in der bei richtigem gegenseitigen Abstand und auf richtigem Höhenniveau bezüglich der Wirbelsäulenerstreckung die endgültige Festlegung der Verbindungseinrichtung auf die starre Kopplung durch Festziehen der Spannschrauben 20a, 20b erreicht wird.

Wie aus den vorgenannten Möglichkeiten zu erkennen, ist eine Verbindung der Stützstangen 40a, 40b einfach zu handhaben und die Verbindungseinrichtung kommt mit wenigen Bauteilen aus.

Wie ebenfalls für den Fachmann erkennbar ist, ist die Konstruktion der Aufnahme 10 der Kopplung nicht auf die der beispielhaft dargestellten Ausführungsform eingeschränkt.

## Patentansprüche

1. Verbindungseinrichtung (100) zur Verbindung zweier längs eines Wirbelsäulenabschnitts gelegten oder zu legenden Stützstangen (40a, 40b) einer Wirbelsäulenstützvorrichtung,
mit einer Querstange (30), einer die Querstange mit einer ersten der Stützstangen koppelnden ersten Kopplung (10a, 20a) und einer die Querstange mit der zweiten der Stützstangen koppelnden zweiten Kopplung (10b, 20b), bei der
wenigstens eine der Kopplungen einen die Stützstange mit einer Klemmkraft einklemmenden Klemmbereich (16, 17) und Kraftbeaufschlagungsmittel zur Erzeugung einer die Klemmkraft bewirkenden Axialkraft (Fₐ) aufweist, in Form einer axialen Spannschraube (20a; 20b), durch deren Einschrauben die Axialkraft bedingt wird, wobei die Axialkraft durch die Querstange geleitet wird,
**dadurch gekennzeichnet, dass** ein federnder Arm (16) zusammen mit einer dem federnden Arm gegenüberliegenden Seitenhalterung (17) den Klemmbereich bildet und dass eine auf der der Spannschraube abgewandten Seite der Querstange gebildete Auflage für die Querstange ohne Beaufschlagung durch die Axialkraft im Bereich des federnden Arms axial auf einem höheren Niveau (Z16) liegt als an der gegenüberliegenden Seitenhalterung (17), so dass die durch die Querstange geleitete Axialkraft zunächst auf die Auflagefläche des federnden Arms wirkt.

2. Verbindungseinrichtung nach Anspruch 1, bei der der Klemmbereich auf die Stützstange aufclipsbar ist.

3. Verbindungseinrichtung nach Anspruch 1 oder 2, bei der eine federnde Lagerung des Federarms durch eine Materialschwächung (67) der Kopplung gefördert ist.

4. Verbindungseinrichtung nach Anspruch 3, bei der im Axialschnitt gesehen die Materialschwächung bezüglich der Spannschraube asymmetrisch gebildet ist.

5. Verbindungseinrichtung nach einem der vorhergehenden Ansprüche, mit einem einnehmbaren ersten Positionierzustand, in dem die Querstange in den Kopplungen durch einen Vorspannungszustand der Kraftbeaufschlagungsmittel auf Positionierabstand der Stützstangen gehalten ist.

6. Verbindungseinrichtung nach Anspruch 5, bei der der Positionierzustand einstellbar änderbar ist.

7. Verbindungseinrichtung nach Anspruch 5 oder 6, mit einem einnehmbaren Sicherungszustand, in dem die Klemmkraft noch Bewegungen der Kopplung entlang der Stützstange, aber nicht mehr ihr Abheben von der Stützstange erlaubt.

8. Verbindungseinrichtung nach Anspruch 7, mit einem einnehmbaren Zustand starrer Kopplung zwischen Stütz- und Querstangen, bewirkbar durch volle Beaufschlagung der Kraftbeaufschlagungsmittel, insbesondere durch ein Festziehen der Spannschraube(n).

9. Verbindungseinrichtung nach einem der vorhergehenden Ansprüche, bei dem die andere der ersten und zweiten Kopplung ebenfalls die Merkmale der vorangegangenen Ansprüche aufweist.

10. Verbindungseinrichtung nach Anspruch 9, bei dem die erste und zweite Kopplung identisch ausgeführt sind.

11. Verbindungseinrichtung nach einem der vorhergehenden Ansprüche, bei der die Axialrichtung zur Erstreckungsrichtung der Querstange, die Axialrichtung zur Erstreckungsrichtung der ersten und/oder zweiten Stützstange und/oder die Erstreckungsrichtung der Querstange zu der der ersten und/oder zweiten Stützstange orthogonal verläuft.

12. Sortiment mit einer Verbindungseinrichtung nach einem der vorhergehenden Ansprüche und wenigstens zwei Querstangen unterschiedlicher Länge und/oder Querabmessung.

13. Sortiment, insbesondere nach Anspruch 12, mit wenigstens einer, bevorzugt wenigstens zwei Verbindungseinrichtungen gemäß einem der Ansprüche 1 bis 11, bei dem wenigstens zwei einer oder zwei Verbindungseinrichtungen zugeordnete Kopplungen Klemmbereiche aufweisen, die zum Einklemmen von Stützstangen unterschiedlicher Querabmessungen ausgelegt sind.

14. Kopplung einer Verbindungseinrichtung nach einem der Ansprüche 1 bis 11 mit der zur Leitung der Axialkraft durch die Querstange ausgelegten Struktur.

15. Herrichten einer Verbindungseinrichtung nach einem der Ansprüche 1 bis 11, aus biokompatiblem Material, für ihren bevorstehenden Einsatz der Schaffung einer Querverbindung zwischen zwei Stützstangen einer Wirbelsäulenstützvorrichtung.

16. Wirbelsäulenstützvorrichtung mit zwei Stützstangen und eine diese verbindenden Verbindungseinrichtung nach einem der Ansprüche 1 bis 11.

## Claims

1. A connector (100) for connecting two support rods (40a, 40b) of a spinal column support device, which are placed or are to be placed along a spinal column portion, said connector comprising:
a transverse rod (30), a first coupling (10a, 20a) coupling the transverse rod to a first one of the support rods and a second coupling (10b, 20b) coupling the transverse rod to the second one of the support rods,
wherein at least one of the couplings has a clamping region (16, 17) that clamps the support rod with a clamping force, and has a force application means for generating an axial force (Fₐ) that creates the clamping force, said force application means being an axial clamping screw (20a; 20b) which causes said axial force when screwed in wherein the axial force is conducted through the transverse rod,
**characterised in that** a resilient arm (16) together with a side bracket (17) opposite the resilient arm form the clamping region, and **in that** a support for the transverse rod formed on the side of the transverse rod facing away from the clamping screw is, when not subjected to the axial force, situated in the region of the resilient arm axially at a higher level (Z16) than on the side bracket (17) opposite, such that the axial force conducted through the transverse rod first acts on the support surface of the resilient arm.

2. The connector according to claim 1, wherein the clamping region can be clipped onto the support rod.

3. The connector according to claim 1 or 2, wherein a resilient mounting of the resilient arm is facilitated by a material weakening (67) of the coupling.

4. The connector according to claim 3, wherein, as seen in an axial cross-sectional view, the material weakening is formed asymmetrically in relation to the clamping screw.

5. The connector according to one of the preceding claims, which is able to adopt a first positioning state, in which the transverse rod is held in the couplings at a positioning distance from the support rods by means of a preloaded state of the force application means.

6. The connector according to claim 5, wherein the positioning state is adjustably variable.

7. The connector according to claim 5 or 6, which is able to adopt a securing state, in which the clamping force still allows movements of the coupling along the support rod but no longer allows said coupling to lift off from the support rod.

8. The connector according to claim 7, which can adopt a state of rigid coupling between the support and transverse rods, which can be created by full application of the force application means, in particular by tightening the one or more clamping screws.

9. The connector according to one of the preceding claims, wherein the other of the first and second coupling also has the features of the preceding claims.

10. The connector according to claim 9, wherein the first and second coupling are identical.

11. The connector according to one of the preceding claims, wherein the axial direction runs orthogonally to the direction of extent of the transverse rod, the axial direction runs orthogonally to the direction of extent of the first and/or second support rod, and/or the direction of extent of the transverse rod runs orthogonally to that of the first and/or second support rod.

12. An assortment comprising a connector according to one of the preceding claims and at least two transverse rods with different lengths and/or transverse dimensions.

13. An assortment, in particular according to claim 12, comprising at least one, preferably at least two connectors according to claims 1 to 11, wherein at least two couplings associated with one or two connectors have clamping regions configured to clamp support rods with different transverse dimensions.

14. A coupling of a connector according to one of claims 1 to 11, having the structure configured to conduct the axial force through the transverse rod.

15. The preparation arrangement of a connector according to one of claims 1 to 11, made of biocompatible material, for its upcoming use to create a transverse link between two support rods of a spinal column support device.

16. A spinal column support device comprising two support rods and a connector according to claims 1 to 11 that connects said support rods.

## Revendications

1. Dispositif de liaison (100) pour relier deux tiges de support (40a, 40b) d'un dispositif de support de colonne vertébrale posées ou à poser le long d'un tronçon de colonne vertébrale, comportant :
une tige transversale (30), un premier coupleur (10a, 20a) qui couple la tige transversale à une première des tiges de support, et un deuxième coupleur (10b, 20b) qui couple la tige transversale à la deuxième des tiges de support ;
au moins un des coupleurs présentant une zone de serrage (16, 17) qui serre la tige de support avec une certaine force de serrage, et des moyens d'application de force destinés à générer une force axiale (Fₐ) qui engendre ladite force de serrage, sous la forme d'une vis de serrage axial (20a ; 20b) dont le vissage provoque la force axiale, ladite force axiale étant conduite à travers la tige transversale,
**caractérisé en ce qu'**un bras élastique (16) forme, conjointement avec un support latéral (17) opposé audit bras élastique, ladite zone de serrage et en ce l'appui destiné à la tige transversale et formée du côté de la tige transversale qui est détourné de la vis de serrage est situé, en l'absence de sollicitation par la force axiale, à proximité du bras élastique à un niveau (Z16) axialement plus élevé que sur le support latéral opposé (17), de telle manière que la force axiale conduite à travers la tige transversale agit d'abord sur la surface de contact du bras élastique.

2. Dispositif de liaison selon la revendication 1, dans lequel la zone de serrage est enclipsable sur la tige de support.

3. Dispositif de liaison selon la revendication 1 ou 2, dans lequel un montage élastique du bras élastique est favorisé par un affaiblissement matériel (67) du coupleur.

4. Dispositif de liaison selon la revendication 3, dans lequel, vu en coupe axiale, l'affaiblissement matériel est formé de manière asymétrique par rapport à la vis de serrage.

5. Dispositif de liaison selon l'une des revendications précédentes, susceptible de prendre un premier état de positionnement dans lequel la tige transversale est maintenue dans les coupleurs, du fait d'un état de précontrainte des moyens d'application de force, selon un écart de positionnement des tiges de support.

6. Dispositif de liaison selon la revendication 5, dans lequel l'état de positionnement peut être modifié de manière réglable.

7. Dispositif de liaison selon la revendication 5 ou 6, susceptible de prendre un état de fixation dans lequel la force de serrage autorise toujours les déplacements du coupleur le long de la tige de support mais ne permet plus son retrait de la tige de support.

8. Dispositif de liaison selon la revendication 7, susceptible de prendre un état de couplage rigide entre les tiges de support et la tige transversale, pouvant être réalisé du fait de la complète sollicitation des moyens d'application de force, notamment par serrage à fond de la ou des vis de serrage.

9. Dispositif de liaison selon l'une des revendications précédentes, dans lequel l'autre des premier et deuxième coupleurs présente également les caractéristiques des revendications précédentes.

10. Dispositif de liaison selon la revendication 9, dans lequel les premier et deuxième coupleurs sont de conception identique.

11. Dispositif de liaison selon l'une des revendications précédentes, dans lequel la direction axiale est orthogonale à la direction d'étendue de la tige transversale, la direction axiale est orthogonale à la direction d'étendue de la première et/ou deuxième tige de support et/ou la direction d'étendue de la tige transversale est orthogonale à celle de la première et/ou deuxième tige de support.

12. Ensemble comportant un dispositif de liaison selon l'une des revendications précédentes et au moins deux tige transversales de longueurs et/ou dimensions transversales différentes.

13. Ensemble, notamment selon la revendication 12, comportant au moins un, de préférence au moins deux dispositifs de liaison selon l'une des revendications 1 à 11, et dans lequel au moins deux coupleurs associés à un ou deux dispositifs de liaison présentent des zones de serrage conçues pour le serrage de tiges de support de différentes dimensions transversales.

14. Coupleur d'un dispositif de liaison selon l'une des revendications 1 à 11 présentant la structure conçue pour conduire l'effort axial à travers la tige transversale.

15. Préparation d'un dispositif de liaison selon l'une des revendications 1 à 11, en matériau biocompatible, en vue de son utilisation prochaine de réalisation d'une liaison croisée entre deux tiges de support d'un dispositif de support de colonne vertébrale.

16. Dispositif de support de colonne vertébrale comportant deux tiges de support et un dispositif de liaison selon l'une des revendications 1 à 11 qui relie ces dernières.
